(19) Europäisches Patentamt
European Patent Office
Office européen des brevets

(11) EP 1 000 940 A1

(12) **EUROPEAN PATENT APPLICATION**

(43) Date of publication:
**17.05.2000 Bulletin 2000/20**

(51) Int Cl.[7]: **C07D 311/72**

(21) Application number: **99121898.3**

(22) Date of filing: **05.11.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **11.11.1998 EP 98121457**

(71) Applicant: **F. HOFFMANN-LA ROCHE AG**
**4070 Basel (CH)**

(72) Inventors:
- **Bonrath, Werner**
  **79115 Freiburg (DE)**
- **Wang, Shaoning**
  **4056 Basel (CH)**

(54) **Process for manufacturing d,l-alpha-tocopherol**

(57) A process for the manufacture of d,1-α-tocopherol by the catalyzed condensation of trimethylhydroquinone with isophytol comprises carrying out the condensation in the presence of bis-(trifluoromethylsulphonyl)amine $[HN(SO_2CF_3)_2]$ or a metal salt thereof of the formula Met $[N(SO_2CF_3)_2]_n$ (I), wherein Met signifies a metal atom selected from the group of boron, magnesium, aluminium, silicon, scandium, titanium, vanadium, manganese, iron, cobalt, nickel, copper, zink, yttrium, zirconium, rhodium, palladium, silver, tin, lanthanum, cerium, neodymium, praseodymium, europium, dysprosium, ytterbium, hafnium, platinum and gold and n signifies the corresponding valency (1, 2, 3, or 4) of the metal atom Met, as the catalyst and in supercritical carbon dioxide or nitrous oxide as the solvent. A further solvent (cosolvent) may be used, which is a lower aliphatic alkanol, ketone or hydrocarbon. The product of the process is the most active member of the vitamin E group.

EP 1 000 940 A1

Printed by Jouve, 75001 PARIS (FR)

## Description

The present invention is concerned with a novel process for the manufacture of d,l-α-tocopherol by the catalyzed condensation of trimethylhydroquinone with isophytol in a solvent. As is known, d,l-α-tocopherol is a diastereomer mixture of 2,5,7,8-tetramethyl-2-(4',8',12'-trimethyl-tridecyl)-6-chromanol (α-tocopherol), which is the most active and industrially most important member of the vitamin E group.

Many processes for the manufacture of d,l-α-tocopherol by the condensation of trimethylhydroquinone (TMHQ) with isophytol (IP) in the presence of various catalysts or catalyst systems and in various solvents are described in the literature. These processes go back to the work of Karrer et al., Bergel et al. as well as Smith et al. [see Helv. Chim. Acta 21, 520-525 (1938), Nature 142, 36 (1938) and, respectively, Science 88, 37-38 (1938) and J. Am. Chem. Soc. 61, 2615-2618 (1939)]. While Karrer et al. carried out the synthesis of d,l-α-tocopherol from TMHQ and phytyl bromide in the presence of anhydrous zinc chloride ($ZnCl_2$; a Lewis acid), not only Bergel et al. but also Smith et al. used TMHQ and phytol as starting materials. In the following years modifications, e.g. alternative solvents and Lewis acids, were developed. From the work of Karrer et al. there was developed in the year 1941 a technically interesting process for the manufacture of d,l-α-tocopherol which was based on the condensation of TMHQ with IP in the presence of the catalyst system $ZnCl_2$/hydrochloric acid (HCl) (US Patent 2 411 969). Later publications, e.g. Japanese Patent Publications (Kokai) 54380/1985, 64977/1985 and 226979/1987 [Chemical Abstracts (C.A.) 103, 123731s (1985), C. A. 103, 104799d (1985) and, respectively, C.A. 110, 39217r (1989)], describe this condensation in the presence of zinc and $ZnCl_2$ and a Bronsted (protonic) acid, such as a hydrohalic acid, e.g. HCl, trichloroacetic acid, acetic acid and the like, especially $ZnCl_2$/HCl, as the catalyst system. Disadvantages of these and further published processes featuring $ZnCl_2$ in combination with a Bronsted acid are the corrosive properties of the acids and the contamination of the waste water with zinc ions as a result of the relativelly large amount of $ZnCl_2$ required for the catalysis.

The manufacture of d,l-α-tocopherol by the reaction of TMHQ with phytyl chloride, phytol or isophytol in the presence of boron trifluoride ($BF_3$) or its etherate ($BF_3 \cdot Et_2O$) is described in German Patents 960720 and 1015446 as well as in US Patents 3 444 213 and 4 634 781. However, $BF_3$ too has corrosive properties.

Also, the condensation of TMHQ with IP or another phytyl derivative in the presence of a Lewis acid, e.g. $ZnCl_2$, $BF_3$ or aluminium trichloride ($AlCl_3$), a strong acid, e.g. HCl, and an amine salt as the catalyst system is described in European Patent Publication (EP) 100471. In an earlier patent publication, Deutsche Offenlegungsschrift (DOS) 2606830, the IP or phytol is pretreated with ammonia or an amine before the condensation with TMHQ in the presence of $ZnCl_2$ and an acid is effected. In both cases corrosion problems occur.

A further interesting method for the manufacture of d,l-α-tocopherol from TMHQ and IP comprises using an isolated TMHQ-$BF_3$ or -$AlCl_3$ complex as the catalyst and a solvent mixture featuring a nitro compound (DOS 1909164). This process avoids to a large extent the formation of undesired byproducts because it involves mild reaction conditions. The yield of d,l-α-tocopherol, based on IP and the use of the solvent mixture methylene chloride/nitromethane, is given as 77%. However, the use of such a solvent mixture is disadvantageous.

The manufacture of d,l-α-tocopherol by the condensation of TMHQ with IP using cation exchange resin complexes of metal ions ($Zn^{2+}$, $Sn^{2+}$ and $Sn^{4+}$) is disclosed in Bull. Chem. Soc. Japan 50, 2477-2478 (1977); amongst other disclosed disadvantages it gives the product in unsatisfactory yields.

The use of macroreticular ion exchangers, e.g. Amberlyst® 15, as the catalyst for the condensation of TMHQ with IP is described in US Patent 3459773.

EP 603 695 describes the manufacture of d,l-α-tocopherol in liquid or supercritical carbon dioxide by the condensation of TMHQ with IP in the presence of acidic catalysts, such as $ZnCl_2$/HCl and ion exchangers.

The condensation in the presence of a catalyst system which consists of iron(II) chloride, metallic iron and HCl gas is described in DOS 2160103 and US Patent 3789086. The formation of less byproducts is advantageous compared with the aforementioned process using $ZnCl_2$/HCl. However, corrosion problems and chloride contamination are equally disadvantageous.

An interesting alternative for the condensation of TMHQ with IP to d,l-α-tocopherol comprises using trifluoroacetic acid or its anhydride as the catalyst (EP 12824). Although in this process the avoidance of HCl is achieved, the alternativ catalyst is relatively expensive.

The use of heteropolytungsten acids as catalysts for the condensation of TMHQ with IP was described for the first time in React. Kinet. Catal. Lett. 47(1), 59-64 (1992). d,l-α-Tocopherol could be obtained in about 90% yield with this process using various solvents.

A further process described in the literature [EP 658 552; Bull. Chem. Soc. Japan 68, 3569-3571 (1995)] for the synthesis of d,l-α-tocopherol is based on the use of a scandium, yttrium or lanthanide fluorosulphonate, nitrate or sulphate, e.g. scandium trifluoromethanesulphonate. With up to about 10% excess of IP this process gives yields up to 98%.

The use of ion exchanged bentonite, montmorillonite or saponite through treatment with e.g. scandium chloride and other metal salts (yttrium, lanthanum, etc.) as the catalyst for the condensation of TMHQ with IP has as a

disadvantage the need for a large amount of catalyst [EP 677 520; Bull. Chem. Soc. Japan 69, 137-139 (1996)].

According to the Examples of EP 694 541 the condensation of TMHQ with IP to α-tocopherol can be achieved in high yields and with a high product purity when such solvents as carbonate esters, fatty acid esters and mixed solvent systems are employed, catalysis being effected by $ZnCl_2$/HCl. Disadvantages in this process are, in addition to the contamination of the waster water by zinc ions, the usual large "catalyst amount" of $ZnCl_2$ used.

According to WO 97/28151 the acid catalysed condensation of TMHQ with IP can be performed in a cyclic carbonate or α-lactone as the solvent. The preferred catalyst is a mixture of ortho boric acid and oxalic, tartaric or citric acid, or boron trifluoride etherate.

Finally, WO 98/21197 describes the manufacture of d,l-α-tocopherol from TMHQ and IP using bis(trifluoromethylsulphonyl)amine or a metal salt thereof optionally together with a strong Bronsted acid, as catalyst in such types of aprotic solvents as aliphatic and cyclic ketones or esters, and aromatic hydrocarbons.

From the foregoing explanations it is evident that most of the previously known processes have considerable disadvantages. Thus, corrosion problems occur in the case of all processes in which such acid catalysts as boron trifluoride are used. Toxicity problems with the boron trifluoride adducts also occur, and when iron or zinc is used there is a contamination of the waste water with the metal ions which is today no longer acceptable. In some processes the formation of undesired byproducts, e.g. phytyltoluene and chlorophytols, is an especially serious problem. Furthermore, the use of organic solvents results in complicated procedures for their removal or recycling during the product isolation.

The object of the present invention is to provide a process for the manufacture of d,l-α-tocopherol by the condensation of trimethylhydroquinone with isophytol in the presence of a catalyst and in a solvent which does not have the disadvantages of previously known procedures. In this respect, it is necessary that the catalyst used does not have a corrosive action, is non-toxic, does not contaminate the environment, catalyzes in small, really catalytic, amounts the desired reaction as selectively as possible and in high yields and should be readily separable and re-usable several times. The solvent should allow more ready isolation of the product than has hitherto been achieved on using organic solvents.

This object of the present invention is achieved by carrying out the condensation of trimethylhydroquinone with isophytol in the presence of a particular amine catalyst and in supercritical carbon dioxide or nitrous oxide. The condensation itself is represented in the following Reaction Scheme, which is presented conventionally:

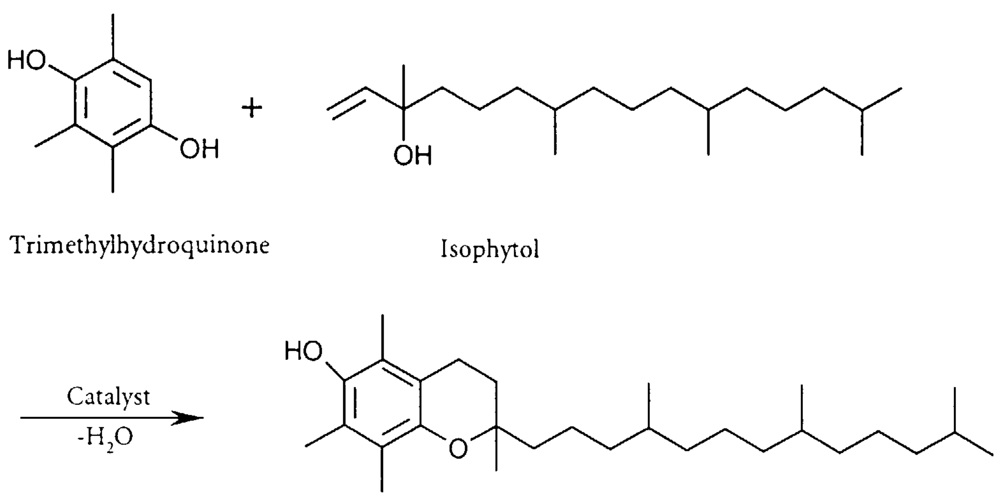


Trimethylhydroquinone

Isophytol

d,l-α-tocopherol

The process in accordance with the invention for the manufacture of d,l-α-tocopherol by the catalyzed condensation of trimethylhydroquinone with isophytol is characterized by carrying out the condensation in the presence of a bis-(trifluoromethylsulphonyl)amine [$HN(SO_2CF_3)_2$] or of a metal salt thereof of the formula

$$Met\,[N(SO_2CF_3)_2]_n \qquad I$$

wherein

Met signifies a metal atom selected from the group of boron, magnesium, aluminium, silicon, scandium, titanium, vanadium, manganese, iron, cobalt, nickel, copper, zinc, yttrium, zirconium, rhodium, palladium, silver, tin, lanthanum, cerium, neodymium, praseodymium, europium, dysprosium, ytterbium, hafnium, platinum and gold

and n signifies the corresponding valency (1, 2, 3 or 4) of the metal atom Met,
as the catalyst and in supercritical carbon dioxide or nitrous oxide as the solvent.

Not only bis-(trifluoromethylsulphonyl)amines, but also some of the metal salts of formula I are known compounds [see, for example, EP 364 340, Japanese Patent Publication (Kokai) 246 338/1995, DOS 19533711, Synlett 1996, 171-172, Synlett 1996, 265-266, Chem. Lett. 1995, 307-308 as well as the literature cited therein]. The new metal salts of formula I can be produced according to methods known per se, namely from the corresponding metal acetates, oxides, hydroxides and alcoholates analogously to the methods for producing the known ones. In the case of the aluminium salt and the zinc salt of bis-(trifluoromethylsulphonyl)amine (of the formula $Al[N(SO_2CF_3)_2]_3$ and $Zn[N(SO_2CF_3)_2]_2$, respectively), these can also be produced from a corresponding alkylmetal or dialkylmetal hydride, e.g. diethylzinc or triethylaluminium, or diisobutylaluminium hydride, respectively.

The metal salts of the bis-(trifluoromethylsulphonyl)amine can be present in monomeric or polymeric form and, accordingly, formula I is intended to embrace all such forms. Further, these catalysts can be used in isolated form or produced in situ.

The expression "supercritical carbon dioxide" is to be understood according to the present invention as indicating carbon dioxide which is present at a temperature and pressure exceeding its critical temperature of 31°C and its critical pressure of 73.8 bar (7.38 MPa). Analogously, "supercritical nitrous oxide" is understood to indicate nitrous oxide which is present at a temperature and pressure exceeding its critical temperature of 36.5°C and its critical pressure of 72.6 bar (7.26 MPa). Both substances are ecologically acceptable and are excellent solvents in the supercritical state for both the reactants and the product of the process according to the present invention.

As well as supercritical carbon dioxide or nitrous oxide, a further solvent (cosolvent) may also be used, which is an organic protic or aprotic solvent. Those organic solvents which especially come into question as the cosolvent are lower aliphatic alkanols, e.g. methanol and ethanol; lower aliphatic ketones, e.g. acetone, isobutyl methyl ketone and diethyl ketone; and lower aliphatic hydrocarbons, e.g. propane. The use of such a cosolvent represents a further aspect of the present invention.

If a cosolvent is employed, the ratio by weight of said cosolvent to supercritical carbon dioxide or nitrous oxide is suitably in the range from 1 : 30 to 1 : 1, preferably in the range from 1.2 : 30 to 9 : 30.

With due consideration to the necessity to effect the condensation of trimethylhydroquinone with isophytol according to the process of the present invention at temperatures and pressures which enable the solvent to be maintained in the supercritical state, the condensation is conveniently effected at temperatures/pressures of about 40 to about 200°C/about 80 to about 270 bar (about 8 to about 27 MPa), preferably at temperatures/pressures of about 110 to about 160°C/about 130 to about 170 bar (about 13 to about 17 MPa). These parameters apply regardless of whether supercritical carbon dioxide or supercritical nitrous oxide is used as the solvent, and these same conditions apply where a cosolvent is also employed.

Furthermore, the molar ratio of trimethylhydroquinone to isophytol present in the reaction mixture for condensation conveniently extends from about 0.8 : 1 to about 1.2 : 1; this molar ratio of trimethylhydroquinone: isophytol is preferably from about 0.9 : 1 to about 1.1 : 1, most preferably about 1 : 1 (as near to equimolar amounts as possible).

The amount of catalyst used is such that the molar ratio of catalyst to the educt (trimethylhydroquinone or isophytol) which is in the lesser molar amount is conveniently about 1 : 1000 to about 1 : 100. In the case of the bis-(trifluoromethylsulphonyl)amine the ratio is preferably about 1 : 500, and in the case of a metal salt of formula I, the ratio is preferably about 1 : 200.

In respect of the amount of supercritical carbon dioxide or nitrous oxide used, in both cases the weight ratio of supercritical carbon dioxide or nitrous oxide to the educt (trimethylhydroquinone or isophytol) which is in the lesser molar amount is suitably about 2 : 1 to about 8 : 1, preferably about 5 : 1 to about 8 : 1, most preferably about 6 : 1. Where a cosolvent is also employed the total weight of supercritical carbon dioxide or nitrous oxide and cosolvent is appropriately increased in accordance with the previously indicated weight ratio of cosolvent to the supercritical solvent.

The actual reaction generally lasts for about 1-5 hours, preferably, about 2-4 hours and most preferably about 2.5-3 hours.

The process in accordance with the invention can be carried out operationally in a very simple manner by heating together the two educts trimethylhydroquinone and isophytol, the catalyst and sufficient carbon dioxide or nitrous oxide, optionally with cosolvent, in an autoclave to maintain the temperature and pressure at the level at which the supercritical state is constantly present. After completion of the condensation reaction, the autoclave is conveniently cooled to room temperature and the pressure released. The crude product can be dissolved in a suitable organic solvent, such as a lower alkanol, e.g. methanol, and the solution concentrated under reduced pressure to isolate the product. At this stage it can be analysed for purity, if desired, by a standard analytical method, e.g. gas chromatography.

The process in accordance with the invention enables the catalyst used to be separated readily and to be re-used several times.

Advantages in the use of the combination of the catalyst and supercritical solvent in the process in accordance with the invention are, in addition to high yields of d,l-α-tocopherol, the avoidance of corrosion, the avoidance of waste water contamination with heavy metal ions, the high selectivity as well as the enabled ready isolation of the produced d,l-α-tocopherol from the mixture after reaction.

The process in accordance with the invention is illustrated by the following Examples:

Examples 1-10

General procedure for batch process

In a stainless steal autoclave (35 ml:"smaller" scale; or 170 ml: "larger" scale) equipped with a magnetic or mechanical stirrer a mixture of trimethylhydroquinone (1.52 g, 9.79 mmol based on 98% pure educt: "smaller" scale; or 7.38 g, 47.52 mmol based on 98% pure educt: "larger" scale), isophytol (approx. 3 g/3.8 ml, approx. 9.6 mmol based on 96% pure educt: "smaller" scale; or 14.92 g/18.4 ml, 46.46 mmol based on 96% pure educt: "larger" scale), a varied amount of catalyst and a varied amount of solvent (supercritical $CO_2$ or $N_2O$) was heated with stirring from room temperature to 150°C and the pressure at said higher temperature measured. The reaction mixture was maintained at 150°C for 3 hours, and thereafter cooled to room temperature again. After releasing the pressure in the autoclave the contents of the autoclave were worked up by dissolution in methanol and evaporation under reduced pressure. The product was then analysed by a standard gas chromatographic method to determine the yield of d,l-α-tocopherol achieved.

The further details of the experimentally performed process and the corresponding yields of d,l-α-tocopherol achieved are presented in the following Table:

| Example | Scale | Catalyst, amount in mg | Solvent, weight in g | Pressure in bar | Percentage yield |
|---|---|---|---|---|---|
| 1 | smaller | $HN(SO_2CF_3)_2$, 10.0 | $CO_2$, 3.7 | 75 | 84.6 |
| 2 | smaller | $HN(SO_2CF_3)_2$, 1.5 | $CO_2$, 3.8 | 75 | 79.8 |
| 3 | smaller | $HN(SO_2CF_3)_2$, 10.0 | $CO_2$, 9.5 | 160 | 86.4 |
| 4 | smaller | $HN(SO_2CF_3)_2$, 10.0 | $CO_2$, 15.2 | 225 | 85.4 |
| 5 | smaller | $HN(SO_2CF_3)_2$, 10.0 | $N_2O$, 10.0 | 155 | 88.3 |
| 6 | larger | $HN(SO_2CF_3)_2$, 48.6 | $N_2O$, 48.6 | 160 | 89.6 |
| 7 | smaller | $AgN(SO_2CF_3)_2$, 10.0 | $CO_2$, 3.8 | 85 | 81.4 |
| 8 | smaller | $AgN(SO_2CF_3)_2$, 65.0 | $CO_2$,11.6 | 160 | 81.8 |
| 9 | smaller | $AgN(SO_2CF_3)_2$, 10.0 | $N_2O$, 10.0 | 160 | 89.0 |
| 10 | larger | $AgN(SO_2CF_3)_2$, 32.4 | $N_2O$, 48.6 | 160 | 83.1 |
| (1 bar = 0.1 MPa) | | | | | |

Example 11

This Example illustrates a semi-batch procedure for the process of the present invention:

A mixture of 15.2 g (98%, 100 mmol) of trimethylhydroquinone, 100.2 mg of $NH(SO_2CF_3)_2$, 10 ml (8.11 g, 96%, 25.22 mmol) of isophytol and 48.6 g $CO_2$ in a 380 ml autoclave equipped with a mechanical stirrer was heated from room temperature to 150°C, after which the pressure was 85 bar (8.5 MPa). Further isophytol (27 ml, 21.9 g, 68.2 mmol) was added within 85 minutes and the reaction mixture was stirred for a further 90 minutes. After completion of the reaction the autoclave was cooled to room temperature. The reaction mixture was then collected and concentrated in vacuo. The crude product was analyzed by gas chromatography, which indicated that a 84.23% yield of d,l-α-tocopherol had been obtained.

## Claims

1. A process for the manufacture of d,l-α-tocopherol by the catalyzed condensation of trimethylhydroquinone with isophytol, which process is characterized by carrying out the condensation in the presence of a bis-(trifluoromethylsulphonyl)amine) [$HN(SO_2CF_3)_2$] or of a metal salt thereof of the formula

$$Met\ [N(SO_2CF_3)_2]_n \qquad I$$

wherein

Met signifies a metal atom selected from the group of boron, magnesium, aluminium, silicon, scandium, titanium, vanadium, manganese, iron, cobalt, nickel, copper, zinc, yttrium, zirconium, rhodium, palladium, silver, tin, lanthanum, cerium, neodymium, praseodymium, europium, dysprosium, ytterbium, hafnium, platinum and gold

and n signifies the corresponding valency (1, 2, 3 or 4) of the metal atom Met,
as the catalyst and in supercritical carbon dioxide or nitrous oxide as the solvent.

2. A process according to claim 1, wherein a further solvent (cosolvent) is used, which is a lower aliphatic alkanol, a lower aliphatic ketone or a lower aliphatic hydrocarbon.

3. A process according to claim 2, wherein the cosolvent is methanol, ethanol, acetone, isobutyl methyl ketone, diethyl ketone or propane.

4. A process according to any one of claims 1 to 3, wherein the condensation is effected at a temperature of about 40 to about 200°C and a pressure of about 80 to about 270 bar (about 8 to about 27 MPa), preferably at a temperature of about 110 to about 160°C and a pressure of about 130 to about 170 bar (about 13 to about 17 MPa).

5. A process according to any one of claims 1 to 4, wherein the molar ratio of trimethylhydroquinone to isophytol in the reaction mixture is from about 0.8 : 1 to about 1.2 : 1, preferably about 0.9 : 1 to about 1.1 : 1, most preferably about 1:1.

6. A process according to any one of claims 1 to 5, wherein the molar ratio of catalyst to trimethylhydroquinone or isophytol, whichever is in the lesser amount, is about 1 : 1000 to about 1 : 100.

7. A process according to any one of claims 1 to 6, wherein the weight ratio of supercritical carbon dioxide or nitrous oxide to trimethylhydroquinone or isophytol, whichever is in the lesser amount, is about 2 : 1 to about 8 : 1, preferably about 5 : 1 to about 8 : 1.

European Patent Office

# EUROPEAN SEARCH REPORT

Application Number
EP 99 12 1898

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,Y | WO 98 21197 A (F. HOFFMANN-LA ROCHE AG) 22 May 1998 (1998-05-22) * claims 1-8 * --- | 1-11 | C07D311/72 |
| D,Y | EP 0 658 552 A (EISAI CO., LTD.) 21 June 1995 (1995-06-21) * claims 1-7 * --- | 1-11 | |
| Y | PATENT ABSTRACTS OF JAPAN vol. 009, no. 309, 12 May 1985 (1985-05-12) & JP 60 149582 A (MITSUI PETROCHEM. IND. K. K.), 7 August 1985 (1985-08-07) * abstract * --- | 1-11 | |
| Y | K. ISHIHARA ET AL.: "Practical Synthesis of (+/-)-alpha-Tocopherol. Trifluoromethanesulfonimide as an Extremely Active Brönsted Acid Catalyst for the Condensation of Trimethylhydroquinone with Isophytol" SYNLETT, no. 11, 1996, pages 1045-1046, XP002064088 * table 2 * ----- | 1-11 | TECHNICAL FIELDS SEARCHED (Int.Cl.7) C07D |

The present search report has been drawn up for all claims

| Place of search | Date of completion of the search | Examiner |
|---|---|---|
| MUNICH | 18 January 2000 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 12 1898

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2000

| Patent document cited in search report | Publication date | Patent family member(s) | Publication date |
|---|---|---|---|
| WO 9821197 A | 22-05-1998 | EP 0937055 A | 25-08-1999 |
| | | US 5908939 A | 01-06-1999 |
| EP 658552 A | 21-06-1995 | JP 7224054 A | 22-08-1995 |
| | | CA 2137481 A | 15-06-1995 |
| | | CN 1108254 A | 13-09-1995 |
| | | DE 69417527 D | 06-05-1999 |
| | | DE 69417527 T | 26-08-1995 |
| | | US 5532387 A | 02-07-1996 |
| JP 60149582 A | 07-08-1985 | NONE | |

EPO FORM P0459

For more details about this annex : see Official Journal of the European Patent Office, No. 12/82